(19) Europäisches Patentamt
European Patent Office
Office européen des brevets

(11) Numéro de publication: **0 359 649 B1**

(12) **FASCICULE DE BREVET EUROPEEN**

(45) Date de publication de fascicule du brevet: **24.11.93**

(51) Int. Cl.$^5$: **C12P 1/00**

(21) Numéro de dépôt: **89402477.7**

(22) Date de dépôt: **12.09.89**

(54) **Procédé de régénération électrochimique de cofacteurs pyridiniques.**

(30) Priorité: **13.09.88 FR 8811933**

(43) Date de publication de la demande:
**21.03.90 Bulletin 90/12**

(45) Mention de la délivrance du brevet:
**24.11.93 Bulletin 93/47**

(84) Etats contractants désignés:
**AT BE CH DE ES FR GB IT LI NL SE**

(56) Documents cités:

**JOURNAL OF BIOTECHNOLOGY, vol. 1, 1984, pages 95-109, Elsevier SciencePublishers B.V.; J. BADER et al.: "Unconventional and effective methods for theregeneration of NAD(P)H in microorganisms or crude extracts of cells"**

**CHEMICAL ABSTRACTS, vol. 99, 1983, page 240, no. 84254r, Columbus, Ohio, US; B.PAYEN et al.: "Use of cytoplasmic hydrogenase from Alcaligenes eutrophus for NADH regeneration" & BIOTECHNOL. LETT. 1983, 5(7),463-8**

(73) Titulaire: **SOCIETE NATIONALE ELF AOUITAINE**
**Tour Elf,**
**2, Place de la Coupole,**
**La Défense 6**
**F-92400 Courbevoie(FR)**

(72) Inventeur: **Durliat, Hélène**
**9 Impasse Brossolette**
**F-31320 Castanet(FR)**
Inventeur: **Comtat, Maurice**
**Résidence du Midi**
**Impasse Delfour**
**F-31400 Toulouse(FR)**
Inventeur: **Seris, Jean-Louis**
**Lot. Parenche No 15**
**Chemin Vignats**
**F-64110 Jurancon(FR)**

(74) Mandataire: **Ohresser, François**
**Société Nationale Elf Aquitaine**
**Division Proprieté Industrielle**
**Tour Elf**
**F-92078 Paris La Défense Cédex 45 (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.6/3.3.1)

BIOTECHNOLOGY & BIOENGINEERING, vol. 27,no. 9, septembre 1985, pages 1277-1281, John Wiley &Sons, Inc., New York, US; T. MATSUNAGA et al.: "Regeneration of NAD(P)H byimmobilized whole cells of clostridium butyricum under hydrogen high pressure"

APPLIED BIOCHEMISTRY AND BIOTECHNOLOGY, vol. 14, 1987, pages 147-197, TheHumana Press Inc.; H. K. CHENAULT et al.: "Regeneration of nicotinamidecofactors for use in organic synthesis"

## Description

La présente invention a pour objet un procédé de régénération électrochimique de cofacteurs pyridiniques utilisés dans les procédés de synthèse enzymatique de produits organiques.

L'étude de l'utilisation des enzymes d'oxydo-réduction en synthèse organique se développe rapidement. Ces procédés de synthèse enzymatique mettent en oeuvre des cofacteurs pyridiniques tels que le NADH (nicotinamide adénine dinucléotide) impliqués dans le mécanisme d'oxydation réduction. Toutefois, la transposition des résultats obtenus en laboratoire à une plus grande échelle nécessite la régénération des cofacteurs pyridiniques beaucoup trop chers pour être utilisés en quantités stoechiométriques. Il semble d'ailleurs que l'absence de méthodes suffisamment efficaces empêche le développement à l'échelle industrielle de procédés de préparation de produits commerciaux. C'est la raison pour laquelle on assiste au développement de nombreux travaux pour la recherche des conditions optimales de régénération de ces cofacteurs. Celle-ci peut être réalisée par voie chimique, enzymatique ou électrochimique. L'analyse des avantages et inconvénients de ces diverses voies montre qu'aucune technique générale de régénération ne donne entière satisfaction et il convient dans chaque cas particulier d'envisager l'optimisation de l'ensemble réaction directe régénération (Applied Biochemistry and Biotechnology, Vol. 14, 1987 pp. 147-197).

Les méthodes de régénération de NADH c'est-à-dire la réduction de la forme $NAD^+$ résultant de processus de réduction du substrat à transformer sont illustrées en figure 1 dans laquelle :

- . S désigne le substrat à transformer
- . P désigne le produit de synthèse à obtenir
- . $E_1$ $E_2$ désignent les enzymes impliquées dans le mécanisme A et B les substrats et produits secondaires impliqués dans la régénération.

Le mécanisme a illustre une régénération chimique; la molécule assurant la régénération réduit directement $NAD^+$ en NADH. L'hydrogène, utilisée sous pression, a été proposée comme une molécule réductrice (Biotechnology and bioengineering, Vol. 7 n° 9 1985, pp. 1277-1281) mais son emploi pose des problèmes de mise en oeuvre et de sécurité.

Le mécanisme b illustre une régénération enzymatique avec une enzyme qui accepte plusieurs substrats: la même enzyme catalyse les réactions de synthèse et de régénération.

Le mécanisme c illustre un processus de synthèse et de régénération mettant chacun en jeu une enzyme et un substrat différents.

Le mécanisme d illustre une régénération électrochimique.

Dans le cas de la réduction de $NAD^+$, les méthodes enzymatiques ont donné les meilleurs résultats. Parmi les systèmes enzymatiques utilisés, on peut citer les couples substrats-enzymes suivants : formiate-formiate déshydrogénase, glucose 6.phosphate-glucose 6.phosphate déshydrogénase, glucose-glucose déshydrogénase, éthanol-alcool déshydrogénase, hydrogène-hydrogénase.

Il a été proposé (Biotechnology letters 1983, 5(7), 463-468) d'utiliser des enzymes telles que l'hydrogénase d'Alcaligenes eutrophus pour réduire $NAD^+$ en NADH par l'hydrogène. Cependant, la stabilité de l'enzyme fixée était très faible, notamment en raison de l'oxygène ou d'oxydants divers, interdisant la mise au point d'un procédé efficace qui, par ailleurs, n'envisage en aucun cas une mise en oeuvre de type électrochimique.

Les procédés électrochimiques semblent attractifs au moins théoriquement parce qu'ils permettent de fixer très facilement la vitesse de régénération par le choix du potentiel d'électrode et d'éviter l'emploi du réactif et de l'enzyme de régénération (fig.1). Par ailleurs, ils offrent la possibilité d'un contrôle facile de la réaction par la mesure de l'intensité d'électrolyse au cours du temps. Toutefois, les avantages sont limités par l'incompatibilité de certains réactifs susceptibles de réagir directement sur l'électrode portée au potentiel de réduction ; la pollution de l'électrode par les produits et les réactifs adsorbables et le manque de sélectivité sont d'autres inconvénients majeurs. Cette dernière difficulté est particulièrement sensible pour la réduction en raison de la formation par transfert monoélectronique de l'intermédiaire radicalaire $NAD^{\cdot}$ susceptible de se dimériser rapidement. Le radical apparaît sur les électrodes quelle que soit leur nature.

Certains essais ont été effectués pour contourner cette difficulté par la modification des surfaces par des médiateurs chimiques fixés ainsi que par l'emploi de médiateurs en solution. Ces tentatives n'ont pas encore permis de trouver une sélectivité suffisante et de ce fait, la réduction électrochimique directe ou indirecte de $NAD^+$ ne s'est pas développée.

La réduction électrochimique de $NAD^+$ a surtout été envisagée sur électrode de mercure sur laquelle apparaît essentiellement le dimère $(NAD)_2$ et NADH dans quelques cas particuliers. L'accent est mis surtout sur l'aspect mécanistique et sur les phénomènes d'adsorption.

Sur électrode de platine nue, la réduction de $NAD^+$ donne un mélange de NADH et $(NAD)_2$ ; elle se produit dans la zone de potentiel où se dégage l'hydrogène gazeux. La réduction est fortement dépendante de l'état de surface de l'électrode.

Il a également été proposé (Journal of Biotechnology, vol. 1 1984 pp. 95-109) d'utiliser des médiateurs comme le méthylviologène, souvent associé à des cellules entières ou à des extraits cellulaires. Dans le mécanisme, le médiateur sert de relais dans le transfert d'électrons entre l'électrode et $NAD^+$ et intervient dans une réaction catalysée par le biosystème. Il faut remarquer que l'apparition d'intermédiaires radicalaires pénalise le rendement ; de plus, la biocompatibilité médiateur biosystème est réduite.

La présente invention a pour but de pallier les inconvénients précédents en proposant un procédé de régénération électrochimique du cofacteur pyridinique, NADH en présence d'une enzyme hydrogénase, la réaction de régénération et la réaction de synthèse étant réalisées dans le même milieu et le même appareillage.

L'invention a pour objet un procédé de régénération électrochimique de cofacteurs pyridiniques utilisés dans les procédés de synthèse enzymatiques par réduction de $NAD^+$ en NADH caractérisé en ce qu'il est réalisé par électrolyse sur une électrode en présence d'une enzyme hydrogénase cytoplasmique.

Les bactéries aérobies capables d'oxyder l'hydrogène comportent en général une hydrogénase soluble, dénommée hydrogénase cytoplasmique, qui réduit $NAD^+$ et une hydrogénase fortement liée sur la membrane, incapable de réduire $NAD^+$, mais dont le rôle est de fournir des électrons pour la génération d'énergie dans une chaîne de transporteurs d'électrons.

On utilisera dans le procédé de l'invention l'hydrogénase cytoplasmique extraite d'une bactérie la comportant. A titre d'exemple de bactéries comportant ce type d'enzyme, on peut citer Nocardia opaca (qui ne comporte pas d'hydrogénase membranaire) Alcaligenes eutrophus, Pseudomonas ruhlandii et Pseudomonas saccharophila ces dernières contenant les deux types d'enzymes. On utilisera de préférence l'hydrogénase cytoplasmique d'Alcaligenes eutrophus.

L'hydrogénase soluble d'Alcaligenes eutrophus possède des centres redox contenant 2 flavines mononucléotides, 2 clusters 4Fe-4S et 2 clusters 2Fe-2S de potentiels normaux apparents (pH 7) respectivement égaux à -0.445 et -0.325 V ainsi que du nickel. Elle diffère des hydrogénases solubles des bactéries anaérobies par son poids moléculaire élevé, sa structure complexe et sa relative tolérance vis-à-vis de l'oxygène.

Ces propriétés font de cette enzyme un cas particulier dans le domaine des hydrogénases en raison des mécanismes complexes de son fonctionnement qui permet son emploi dans le domaine de la biotechnologie pour la régénération de NADH.

Il semble que ce mécanisme de fonctionnement permette un transfert électronique direct électrode-enzyme (hydrogénase) particulièrement favorable à la réduction du $NAD^+$ sans création des radicaux $NAD^·$ précurseurs du $(NAD)_2$.

La flavine de l'hydrogénase joue sans doute un rôle important dans ce transfert en assurant le relais électronique entre l'électrode et les clusters Fe-S qui se réduisent comme l'indique l'évolution des spectres.

Le schéma de régénération électrochimique utilisant l'hydrogénase cytoplasmique est illustré figure 2 dans laquelle :

. Hase désigne : l'hydrogénase d'Alcaligenes eutrophus
. E désigne : l'enzyme qui catalyse la réaction chimique ayant lieu en solution
. S et P désignent : le substrat et le produit participant à la réaction chimique
. C désigne : l'électrode, cathode au niveau de laquelle est engendré l'électron.

La réduction de l'enzyme par électrochimie peut être mise en évidence par spectroscopie au potentiel de -0,65 volt ; la réoxydation s'amorce à - 0,5 volt et se complète à + 0,1 volt.

La mesure de l'activité enzymatique effectuée sur la solution prélevée après plusieurs réductions et réoxydations indique que le transfert électronique n'entraîne pas de perte d'activité. Au contraire dans les premiers instants après l'arrêt de l'électrolyse on observe une légère augmentation de cette valeur.

L'enzyme joue donc un rôle de médiateur d'oxydo-réduction entre l'électrode et $NAD^+$ et aucune dénaturation de $NAD^+$ ou NADH n'apparaît lorsque ces molécules sont mises dans un champ électrique. Par ailleurs, contrairement aux expériences de réduction directe, le dimère n'a jamais été mis en évidence. On peut sans doute expliquer un transfert biélectronique facilité par la formation du complexe enzyme-$NAD^+$ dans lequel le cofacteur est dans la structure la plus favorable pour accepter deux électrons, l'enzyme servant de relai électronique. La formation du dimère n'a pas lieu car l'adsorption de $NAD^+$ ou NAD sur l'électrode est probablement empêchée par une plus forte adsorption de l'enzyme.

Dans le procédé de l'invention on pourra utiliser toute électrode inattaquable c'est-à-dire restant dans son état originel en présence du solvant du milieu, contenant ou non de l'oxygène, et donc ne pouvant former avec ledit milieu des ions susceptibles de passer en solution. A titre d'exemples d'électrodes

utilisables on peut citer les électrodes en métaux précieux or, platine, iridium (ou leurs alliages), les électrodes au carbone, au tungstène ou au nickel. On utilisera de préférence les électrodes en platine.

Les conditions opératoires température pH du procédé de l'invention sont étroitement liées à la réaction de synthèse principale en assurant pour celle-ci la stabilité des enzymes et un rendement maximal. La température sera généralement comprise entre 0 et 40 ° C et le pH compris entre 5 et 10.

Le point sensible de procédé est de bien réguler le potentiel de l'électrolyse. Il sera en général compris entre - 0,5 et - 0,9 volt et de préférence entre - 0,65 et - 0,75 volt. Il importe d'éviter de trop abaisser ce potentiel qui risquerait d'entraîner un dégagement d'hydrogène et donc une augmentation du pH susceptible de dénaturer les enzymes et/ou le substrat et de porter atteinte à la vitesse de réaction.

Le procédé de l'invention est de portée générale, c'est-à-dire, qu'il est utilisable pour une large gamme de réactions d'oxydo-réduction enzymatiques. Cette polyvalence sera illustrée dans les exemples donnés ci-après pour plusieurs réactions différentes.

Les conditions opératoires précises de mise en oeuvre du procédé : potentiel, pH du milieu, teneur, sont déterminées cas par cas selon la réaction enzymatique directe à réaliser et cela par des techniques bien connues.

La régénération bioélectrocatalytique de NADH s'effectue par réduction de $NAD^+$ en présence d'hydrogénase, couplée à une réaction de réduction enzymatique suivant le schéma de la figure 2.

Sur un plan pratique, une étude préliminaire doit être réalisée consistant à vérifier que, lorsque l'électrode est portée au potentiel de réduction de l'hydrogénase et de formation de NADH

- le substrat n'est pas réduit directement
- le substrat et le produit sont stables
- l'enzyme E ne perd pas son activité dans le champ électrique.

Le procédé de l'invention est illustré par les exemples 1 à 3 ci-après visant la régénération de NADH utilisée pour la mise en oeuvre des réactions 1 à 3.

$$1 \quad CH_3-CO-COO^- + NADH + H^+ \underset{a}{\overset{b}{\rightleftarrows}} CH_3-CHOH-COO^- + NAD^+$$

$$2 \quad CH_3-CO-CH_2-CO-SCoA + NADH \underset{a}{\overset{b}{\rightleftarrows}} CH_3-CHOH-CH_2-CO-SCoA + NAD^+$$

$$3 \quad {}^-OOC-CH_2-CH_2-CO-COO^- + NH_4^+ + NADH \underset{a}{\overset{b}{\rightleftarrows}}$$

$$\underset{\phantom{CH_2-CH_2-}NH_2}{{}^-OOC-CH_2-CH_2-CH-COO^-} + NAD^+ + H_2O$$

Ces réactions sont respectivement catalysées par :

- une large lactate déshydrogénase de muscle de lapin (LDH)
- une hydroxyacylcoenzyme A déshydrogénase de coeur de porc (HyACoADH)
- une glutamate déshydrogénase de foie de boeuf (G1DH)

Pour toutes ces expériences, on a utilisé la technique de la spectroélectrochimie en couche mince. La cellule d'électrolyse incluait une électrode en platine placée entre deux parois de verre définissant un volume de milieu réactionnel compris entre 20 et 200 μl, analogue à celui utilisé par De Angélis et Coll. (J. Chem. Educ. 1976, 53,594). Les mesures sont effectuées par spectrophotométrie (mesure de l'absorbance).

Ces expériences de régénération couplées à une réaction enzymatique consistent en des électrolyses à un potentiel déterminé précédemment de manière conventionnelle. Le substrat, le cofacteur et les deux enzymes en solution dans le milieu approprié à la réaction sont introduits dans la couche mince ; après une

réduction de 90 min., la solution est prélevée et la mesure des concentrations du produit et du cofacteur restant est effectuée.

Les solutions électrolytiques sont préparées à partir d'eau distillée et de sels de grande pureté. Les différentes solutions utilisées sont réalisées de la façon suivante :

- pH 6.5 et 7 : solution de phosphate monopotassique et disodique 0.2 M en phosphate ou solution de triéthanolamine 0.05 M, HCl
- pH 8 : solution phosphate 0.2 M
- pH 9 : solution de carbonate de potassium 0.1 M ou solution de glycine 0.6 M, hydrazine 0.5 M
- pH 10 : solution de carbonate de potassium 0.1 M

Les produits biologiques proviennent de la Société Sigma à l'exception de l'hydrogénase cytoplasmique d'Alcaligenes eutrophus, extraite suivant les méthodes classiques (Schneider K et Coll. Biochem. Biophys. Res. Commun 1978, 84,564). La concentration moyenne est de 20 mg/ml avec une activité spécifique égale à 40 U/mg. Les autres enzymes sont de provenances diverses : la glutamate déshydrogénase est extraite de foie de boeuf, l'hydroxyacétylCoA déshydrogénase de coeur de porc, la lactate déshydrogénase de muscle de lapin.

Les différents dosages, spectrophotométriques, sont réalisés en mettant en oeuvre les réactions indiquées ci-dessus et ne nécessitent que quelques microlitres de solution.

- Exemple 1 : lactate et pyruvate : on met en oeuvre la réaction 1 et le dosage spectrophotométrique de NADH à 340 nm. Pour le lactate le pH de la solution est égal à 9 en milieu glycine, la réaction est relativement lente. Le dosage du pyruvate a lieu en milieu phosphate à pH 8 pour lequel la réaction est rapide.
- Exemple 2 : acétoacétylCoA : on utilise pour le dosage du réactif et du produit la réaction 2 favorisée dans le sens a à pH 7 dans le sens b à pH 10 ; dans les deux cas, on peut suivre la disparition ou l'apparition de NADH par spectrophotométrie à la longueur d'onde de 340 nm.
- Exemple 3 : -cétoglutarate : le dosage est réalisé en milieu triéthanolamine ou en milieu phosphate, le pH de la solution étant égal à 7. Sont ajoutés $NH_4^+$ 0.1 M, NADH 0.27 mM, la glutamate déshydrogénase et la disparition de NADH au cours de la réaction 3 est suivie au cours du temps.
- glutamate : la solution a un pH égal à 8.6 ; on utilise la réaction 3 dans le sens b couplée à la réaction NADH + INT + $N^+$ ---> $NAD^+$ + formazan qui favorise le déplacement de l'équilibre 3 dans le sens b (INT est l'iodonitrotétrazolium). Le formazan est dosé spectrophotométriquement à la longueur d'onde de 492 nm.

Les conditions générales de ces essais et les résultats obtenus sont résumés dans le tableau 1 ci-dessous qui concernent la régénération du cofacteur par réduction de $NAD^+$.

TABLEAU 1

| Exemple | Substrat à réduire | $NAD^+$ mM | Hase | Enzyme E | milieu | potentiel V | TTN |
|---|---|---|---|---|---|---|---|
| 1 | pyruvate 6 mM | 0.5 | 12µL | LDH | phosphate pH 8 | - 0.72 | 9 |
| 2 | acétoacétyl-SCoA 4 mM | 0.5 | 20µL | HyCoADH 5 µL | phosphate pH 7 | - 0.68 | 6 |
| 3 | cétoglutarate 8 mM | 0.6 | 20µL | GlDH 1mg/ml | TEA ou phosphate + $NH_4^+$ pH 7 | - 0.68 | 11 |
| | puis ajout 9 mM | 0.4 | | | | | 12 |

TTN = rapport entre les nombres de moles de produit formé et de cofacteur en solution.

Pour ces expériences, le temps d'électrolyse a été normalisé à 90 mn. Le total turnover (TTN), c'est-à-dire le rapport du nombre de mole de produit formé au nombre de mole de cofacteur, a été déterminé à partir du bilan effectué sur les concentrations de substrat et de produit au moment du prélévement, ce qui permet de vérifier que l'enzyme joue son rôle normal dans la réaction étudiée. Les résultats présentés sont la moyenne de 5 à 10 expériences pour chaque substrat. Ils amènent quelques commentaires :

- la valeur du TTN prouve la régénération du cofacteur et son faible niveau ne provient que de la géométrie de la cellule et des conditions expérimentales non optimisées de mise en oeuvre de la régénération.
- $NAD^+$ se retrouve en totalité à l'état natif à la fin de l'expérience, sans traces détectables de dimère $(NAD)_2$. Le procédé peut s'étendre à d'autres réactions enzymatiques et se révèle original car il semble que ce soit le premier exemple d'emploi d'une enzyme comme relai électrochimique direct entre une électrode et $NAD^+$. Sa mise en oeuvre passe par une optimisation du réacteur électrochimique qui, en particulier, doit être en atmosphère inerte. D'autre part, la précision du contrôle du potentiel de l'électrode de travail fait de la méthode électrochimique un outil plus simple et plus sélectif que la réduction par l'hydrogène moléculaire qui utiliserait la même enzyme.

Dans le processus industriel, le cofacteur est généralement ajouté au milieu sous la forme oxydée $NAD^+$.

Il est alors très avantageux d'ajouter au milieu, à côté de $NAD^+$ des traces de NADH, ce qui permet de mieux initier la réaction, et de maintenir ce léger excès de NADH durant le processus en continu.

## Revendications

1. Procédé de régénération par réduction d'un cofacteur pyridinique de type $NAD^+$/NADH utilisé dans une réaction enzymatique de synthèse organique caractérisé en ce qu'il est réalisé au sein du milieu réactionnel au moyen d'une électrode en présence d'une enzyme hydrogénase cytoplasmique.

2. Procédé selon la revendication 1 caractérisé en ce que l'enzyme est l'hydrogénase cytoplasmique d'Alcaligenes eutrophus.

3. Procédé selon une des revendications 1 ou 2 caractérisé en ce que l'électrode est constitué d'un métal précieux.

4. Procédé selon la revendication 3 caractérisé en ce que l'électrode est en platine.

5. Procédé selon l'une des revendications 1 à 4 caractérisé en ce que le potentiel de l'électrolyse est compris entre - 0,5 et - 0,9 volt.

6. Procédé selon la revendication 5 caractérisé en ce que le potentiel est compris entre - 0,65 et - 0,75 volt.

7. Procédé selon l'une des revendications 1 à 6 caractérisé en ce que le pH est compris entre 5 et 10.

8. Procédé selon l'une des revendications 1 à 7 caractérisé en ce que des traces de NADH sont ajoutés au milieu en même temps que le cofacteur sous forme de $NAD^+$.

## Claims

1. Method of regenerating a pyridic cofactor of the $NAD^+$/NADH type by reduction, the cofactor being used in an enzymatic organic synthesis reaction, characterized in that it is carried out within the reaction medium by means of an electrode, in the presence of a cytoplasmic hydrogenase enzyme.

2. Method according to Claim 1, characterized in that the enzyme is the cytoplasmic hydrogenase of Alcaligenes eutrophus.

3. Method according to Claim 1 or Claim 2, characterized in that the electrode is made of a precious metal.

4. Method according to Claim 3, characterized in that the electrode is of platinum.

5. Method according to any one of Claims 1 to 4, characterized in that the electrolysis potential is between 0.5 and 0.9 volts.

6. Method according to Claim 5, characterized in that the potential is between 0.65 and 0.75 volts.

7. Method according to any one of Claims 1 to 6, characterized in that the pH is between 5 and 10.

8. Method according to any one of Claims 1 to 7, characterized in that traces of NADH are added to the medium, at the same time as the cofactor, in the form of $NAD^+$.

## Patentansprüche

1. Ein in einer enzymatischen Reaktion zur organischen Synthese verwendetes Verfahren zur Regenerierung durch Reduktion eines Pyridinkofaktors vom Typ $NAD^+$/NADH, dadurch gekennzeichnet, daß es in einem Reaktionsmedium mit einer Elektrode in Gegenwart eines cytoplasmischen Hydrogenaseenzyms durchgeführt wird.

2. Verfahren nach Anspruch 1, dadurch gekennzeichnet, daß das Enzym die cytoplasmische Hydrogenase eines Alcaligenes eutrophus ist.

3. Verfahren nach einem der Ansprüche 1 oder 2, dadurch gekennzeichnet, daß die Elektrode aus einem Edelmetall ist.

4. Verfahren nach Anspruch 3, dadurch gekennzeichnet, daß die Elektrode aus Platin ist.

5. Verfahren nach einem der Ansprüche 1 bis 4, dadurch gekennzeichnet, daß das Potential der Elektrolyse zwischen -0,5 und -0,9 Volt beträgt.

6. Verfahren nach Anspruch 5, dadurch gekennzeichnet, daß das Potential zwischen -0,65 und -0,75 Volt beträgt.

7. Verfahren nach einem der Ansprüche 1 bis 6, dadurch gekennzeichnet, daß der pH zwischen 5 und 10 beträgt.

8. Verfahren nach einem der Ansprüche 1 bis 7, dadurch gekennzeichnet, daß die NADH-Spuren dem Medium gleichzeitig mit dem Kofaktor in Form von $NAD^+$ zugegeben werden.

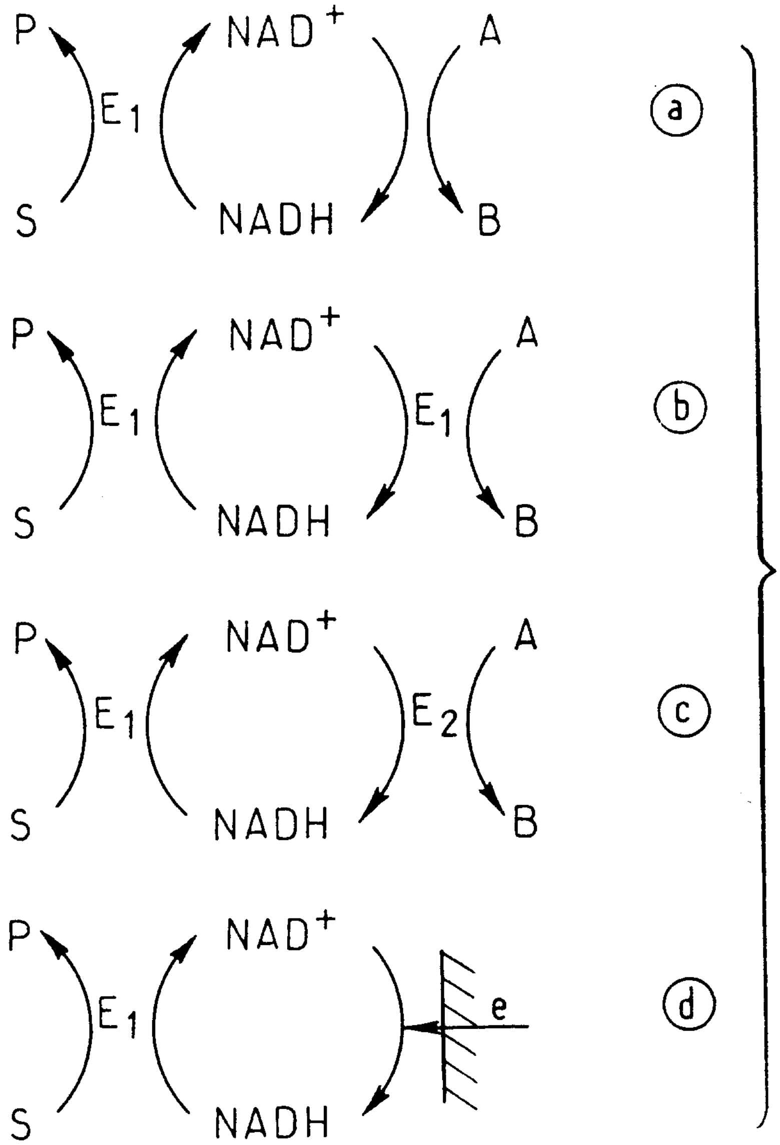
P
NAD+
A
E1
a
S
NADH
B
P
NAD+
A
E1
E1
b
S
NADH
B
P
NAD+
A
E1
E2
c
S
NADH
B
P
NAD+
E1
e
d
S
NADH

FIG_1

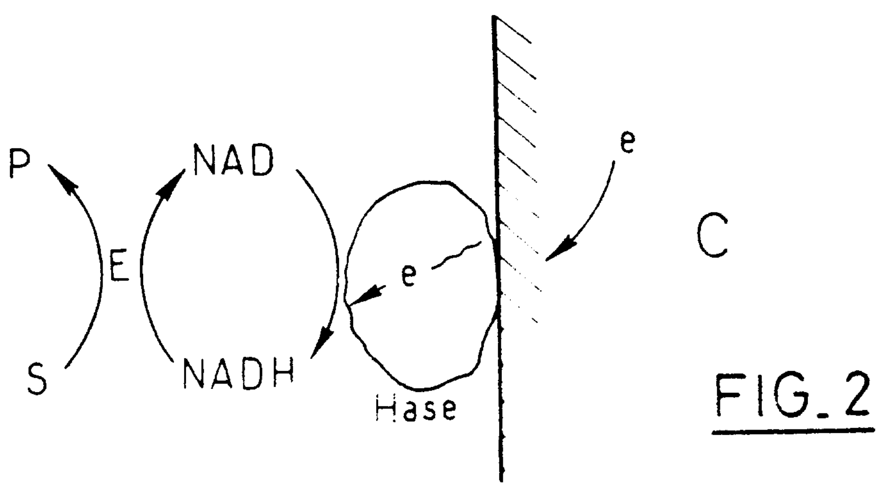
P
NAD
E
e
e
S
NADH
Hase
C

FIG_2